# EUROPEAN PATENT SPECIFICATION

(11) **EP 4 570 197 B1**
(45) Date of publication and mention of the grant of the patent: **09.12.2026**
(21) Application number: 24217759.0
(22) Date of filing: 31.08.2010
(51) Int. Cl.: A61B 17/34, A61B 17/02, A61B 90/00, A61B 17/00

(54) **MULTIFUNCTIONAL SURGICAL ACCESS SYSTEM**
MULTIFUNKTIONALES CHIRURGISCHES ZUGANGSSYSTEM
SYSTÈME D'ACCÈS CHIRURGICAL MULTIFONCTIONNEL

(30) Priority: 31.08.2009 US 238540 P
(43) Date of publication of application: 18.06.2025
(62) Document number(s) of the earlier application(s) in accordance with Art. 76 EPC: 23173591.1 / 4 248 881; 19213801.4 / 3 636 183; 10752681.6 / 2 473 124
(73) Proprietor: Applied Medical Resources Corporation, Rancho Santa Margarita, CA 92688 (US)
(72) Inventor: ALBRECHT, Jeremy J, Rancho Santa Margarita, 92688 (US); NGUYEN, Eric, Rancho Santa Margarita, 92688 (US); BECERRA, Matthew M, Rancho Santa Margarita, 92688 (US); JOHNSON, Gary M, Rancho Santa Margarita, 92688 (US); BRUSTAD, John R, Rancho Santa Margarita, 92688 (US); SAKAKINE, Ghassan, Rancho Santa Margarita, 92688 (US)
(74) Representative: Dolleymores

(56) References cited:
- WO-A1-2008/147644
- WO-A2-2004/075730
- WO-A2-2004/075741
- WO-A2-2007/083305
- US-A- 5 460 170
- US-A1- 2003 216 770
- US-A1- 2005 059 865
- US-A1- 2007 088 204
- US-A1- 2007 270 654

## Description

### BACKGROUND

### Technical Field

This application relates to a tissue, wound, retractor of a surgical access system.

### Description of the Related Art

Wound retractors are used to expand or retract a surgical incision or natural orifice. Some wound retractors comprise a polymer film sheath disposed between an inner and outer ring. In use, the inner ring is inserted into a body cavity, such as an abdominal cavity, and the film is anchored to the outer ring under tension. The tensioned film stretches the incision or orifice, thereby improving access to the cavity. The polymer film sheath also lines the incision or opening, thereby protecting the soft tissue from contamination and/or physical damage.

Examples of known wound retractors are shown in the following patent documents bearing publication numbers US 2007/27054 A1, WO 2007/083305 A2 and WO 2008/147644 A1.

### SUMMARY OF THE INVENTION

According to the present invention there is provided a tissue retractor as recited in Claim 1. Further embodiments are provided in the dependent claims.

### BRIEF DESCRIPTION OF THE DRAWINGS

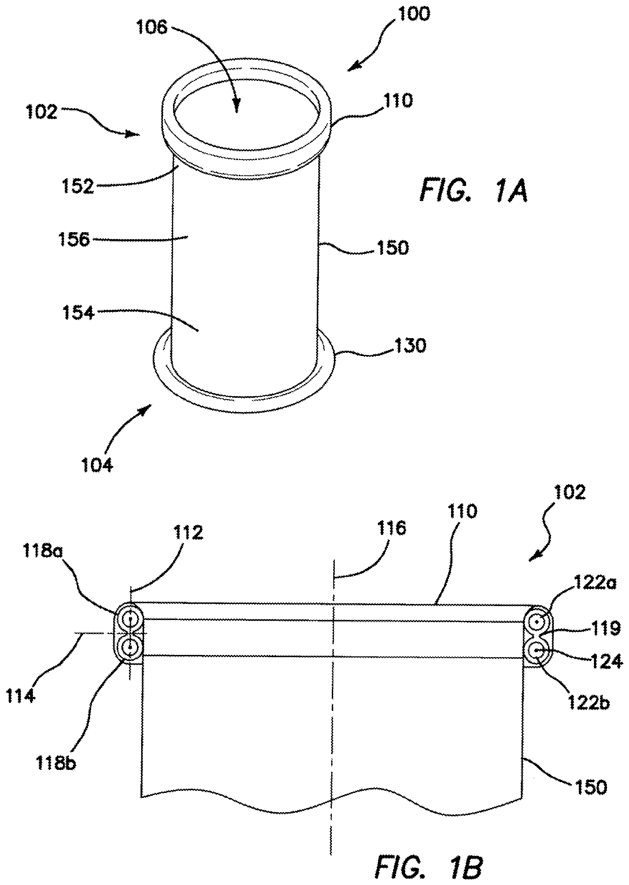

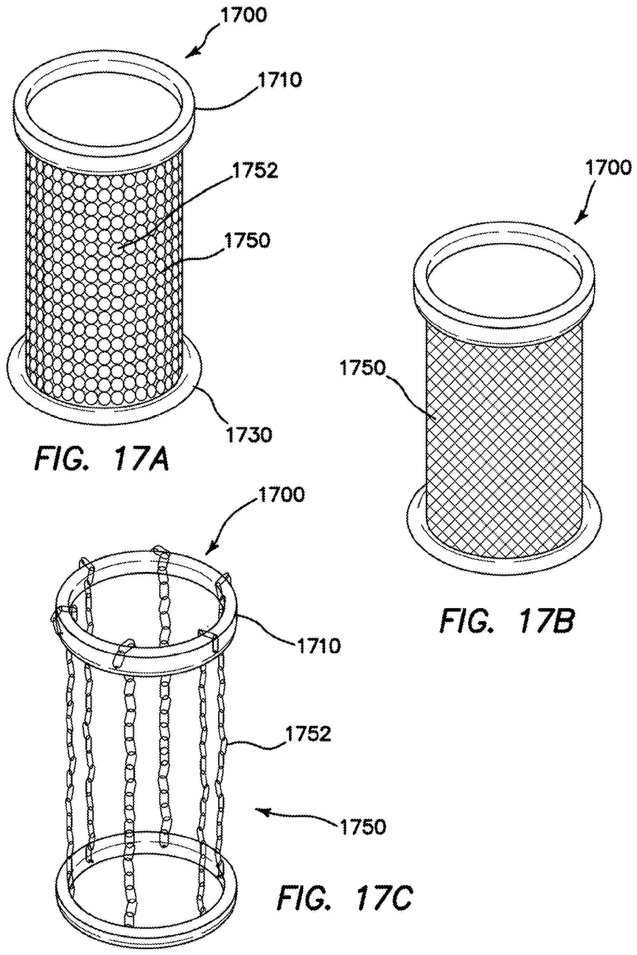

Features of retractors are described below with reference to the figures, where FIGS 1A to 16 disclose features which are not in accordance with the present invention, but which may be helpful to the understanding of a retractor in accordance with the present invention, and where FIGS 17A to 17C disclose embodiments of retractors in accordance with the present invention.
FIG. **1A** is a perspective view of a retractor. FIG. **1B** is a side cross section of a proximal end of the retractor illustrated in FIG. **1A****.**
FIGS 2A to 16 illustrate features of a retractors FIGS. **2A-2K** are cross sections of outer rings.
FIG. **3** is a perspective view of a reshapeable inner ring of a tissue retractor in accordance with the present invention.
FIGS. **4A** and **4B** are perspective views of embodiments of inner rings with thinned edges.
FIG. **5** is a partial cross section of a collapsible inner ring.
FIG. **6** is a detailed view of a proximal end of a retractor comprising a woven fabric sheath.
FIG. **7A** is a perspective view of another retractor.
FIG. **7B** is a side cross section of a proximal end of the retractor illustrated in FIG. **7A****.**
FIG. **7C** is a detail view of an outer ring and sheath of the retractor illustrated in FIG. **2A****.**
FIG. **8A** is a perspective view of an unassembled retractor kit.
FIG. **8B** is a detail view of an inner ring.
FIG. **8C** is a perspective view of another inner ring.
FIG. **9A** is a top view and FIG. **9B** is a side view of an outer ring.
FIG. **10** is a partial side cross section of another retractor.
FIG. **11A** is a perspective view and FIG. **11B** is a detailed view of another retractor.
FIG. **12A** is a perspective view and FIG. **12B** is a detailed view of another retractor.
FIG. **13** is an exploded view of a retractor comprising a shield.
FIG. 14 is a top cross section of a resizable inner ring.
FIG. **15** is a side cross section of another resizable inner ring.
FIG. **16** is a perspective view of an inner anchor.
FIG. **17A** is a side view of an embodiment of a retractor in accordance with the present invention, comprising a metal sheath.
FIG. **17B** is a side view of another embodiment of a retractor not in accordance with the present invention, comprising braided wire.
FIG.**17C** is a side view of another embodiment of a retractor in accordance with the present invention, comprising a metal sheath.

Similar reference numbers refer to similar components.

### DETAILED DESCRIPTION OF CERTAIN EMBODIMENTS

Surgical access systems similar to embodiments disclosed herein are disclosed in U.S. Patent Publication Nos. 2005/0241647 A1, 2007/0185387 A1, 2007/0149859 A1, and 2007/0088202 A1.

FIG. **1A** is a perspective view of a wound retractor **100** of a surgical access system. The retractor **100** comprises a proximal end **102,** a distal end **104,** an instrument access channel **106,** an outer or proximal anchor **110,** and inner or distal anchor **130,** and a flexible sheath **150** extending between the outer anchor **110** and the inner anchor **130.** Both of the outer anchor **110** and the inner anchor **130** are ring-shaped, and consequently, are referred to as an outer ring **110** and an inner ring **130,** respectively. Both of the inner ring **110** and the outer ring **130** shown are generally circular in a top or plan view, but at least one of the outer ring **110** and the inner ring **130** need not be circular, for example, one might be oval, elliptical, D-shaped, and the like. Furthermore, the illustrated outer ring **110** and inner ring **130** generally have the same diameter. Alternatively, the outer ring **110** and the inner ring **130** may independently have different diameters, sizes, and/or shapes. For example, the inner ring **110 may** have a smaller diameter than the outer ring **130,** or the inner ring **110** may have a larger diameter than the outer ring **130.** The flexible sheath **150** adopts the shape of the outer ring **110** and the inner ring **130.** Consequently, the flexible sheath **150** illustrated is generally cylindrical.

he outer ring **110** comprises an annular axis around which the outer ring **110** is rotatable or invertible in a process through which the outer ring **110** is rolled through itself, as will be discussed in greater detail below. Consequently, the outer ring **110** comprises a flexible material. This may comprise one or more polymers, for example flexible engineering plastics, an elastomer, for example a thermoplastic elastomer, a composite, for example a polymer and a reinforcing material. Examples of suitable reinforcing materials include fibers, fabrics, and the like, which comprise at least one of polymer, metal, glass, ceramic, and the like. The outer ring **110** may be molded and/or extruded as a single piece or as a plurality of pieces that are assembled into the outer ring **110.**

FIG. **1B** illustrates a side cross section of the proximal end of the retractor **100.** The cross section of the outer ring **110** comprises a major or longer axis **112** and a minor or shorter axis **114.** The major axis **102** is generally parallel with a longitudinal axis **118** of the outer ring **110,** while the minor axis is generally parallel with a radial axis thereof. The relative positions of the major **112** and minor **114** axes may though be reversed. As best viewed in FIG. **1B****,** the outer ring **110** comprises a plurality of lumens: a first lumen 122*a* disposed on the major axis above the minor axis, and a second lumen **122***b*, disposed on the major axis below the minor axis. Consequently, the first lumen **122a** is disposed above the second lumen **122***b*. The outer ring **110** may alternatively comprise a different number of lumens, for example, one lumen, three lumens, or even more lumens.

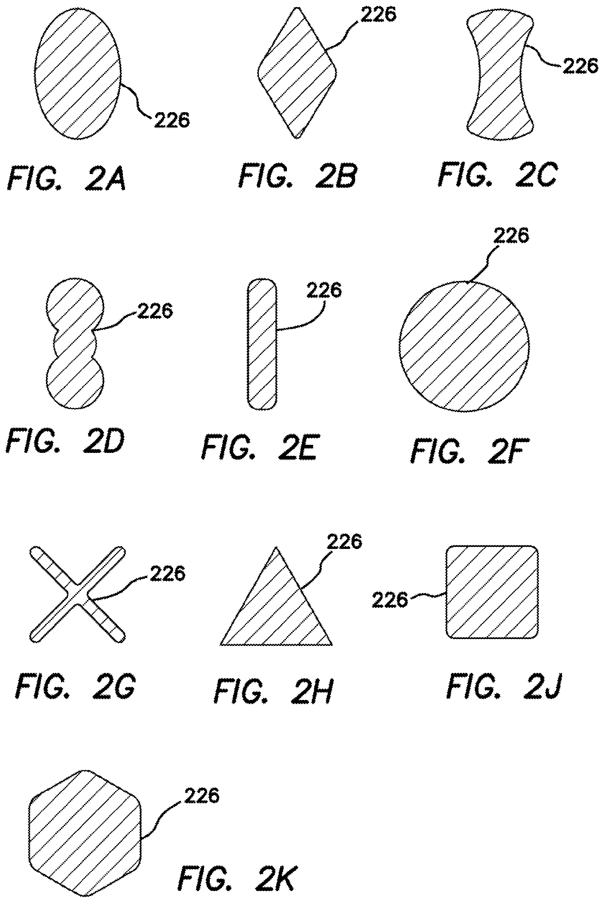

In FIG. **1B****,** the cross-sectional shape of the outer ring **110** is generally a figure-8, or first circle **118***a* and a second circle **118***b* joined by a web **119** extending therebetween. The first lumen **122***a* is disposed in the first circle **118***a*, and the second lumen **122***b* is disposed in the second circle **188***b*. FIGS. **2A-2K****,** illustrate different cross-sectional shapes generally oval or elliptical (FIG. **2A**); diamond-shaped or rhomboid (FIG. **2B**); hourglass or dog bone shaped (FIG. **2C**); snowman-shaped (FIG. **2D**); radially flat (washer-shaped outer ring), longitudinally flat (cylindrical outer ring), or flat at another angle (frustoconical outer ring) (FIG. **2E**); circular (toroidal outer ring) (FIG. **2F**), X-shaped (FIG. **2G**), triangular (FIG. **2H**), square (FIG. **2J**), hexagonal (FIG. **2K**), polygonal, and the like. The outer ring may comprise one or more gripping surfaces **226** that facilitate manually rolling the outer ring around the annular axis thereof. Examples of suitable gripping surfaces include generally flattened surfaces as shown in FIGS. **2A, 2B, 2E, 2H, 2J,** and **2K****;** and concave surfaces as shown in FIGS. **2C, 2D,** and **2G****.** Any of the arrangements illustrated in FIGS. **2A-2K** may optionally comprise one or more circumferential lumens in which wires or hoops are optionally disposed, as discussed below. The outer ring **110** may have a Möbius configuration in which the outer ring **110** is fabricated with a preloaded circumferential torsional stress, for example, by twisting an elongate member followed by joining the ends.

A wire or rod **124** can be disposed in at least one of the first lumen **122***a* and the second lumen **122***b* as illustrated in FIG. **1B****,**in which where the ends of the wire or rod **124** contact or nearly contact each other in the lumen, this is referred to herein as a "split hoop". A split hoop may be in each of the first lumen **112** and the second lumen **114,** or a single split hoop **124** may be used, the split hoop **124** defining the annular axis. Where a plurality of split hoops **124** are present, rotating the outer ring **110** around the annular axis sequentially subjects each split hoop **124** to compression followed by then tension. Here, the split hoop **124** under compression defines the annular axis for that portion of the rotation.

The split hoop(s) **124** are substantially non-compliant under the conditions under which the retractor **100** is used, and this may render the outer ring **110** substantially non-compliant, for example, resisting compression. Alternatively, the split hoop(s) **124** are compliant and the outer ring **110** is also compliant. In some arrangements, the outer ring **110** does not comprise a rod or wire disposed in a lumen thereof. A non-compliant outer ring **110** may facilitate direct coupling of another device to the outer ring **110** for example, a lid, cap, and/or gel cap. Some compliant outer rings **110** may conform to a body surface.

The outer ring **110** may comprise a solid or non-split hoop **124** disposed in a lumen. As discussed above, the solid hoop **124** may define an annular axis around which the outer ring **110** is rotatable. The outer ring **110** may comprise a solid hoop and at least one split hoop. The solid hoop and/or split hoop may maintain the top- or plan-view shape of the outer ring **110,** for example, circular, oval, elliptical, or D-shaped, and/or maintain a side-view profile, for example, flat, curved, or saddle-shaped. The hoop(s) **124** may influence the rotational characteristics of the outer ring **110,** for example, preventing rotation or permitting rotation, as discussed below. The hoop(s) **124** may influence the orientation of the outer ring **110,** for example, with the major axis **112** parallel with the longitudinal axis **116** of the outer ring **110,** as illustrated in FIG. 1B, or with the major axis **112** perpendicular with the longitudinal axis **116.**

In some arrangements, a profile or graph of a potential energy of the outer ring **110** versus rotation around the annular axis over 360° comprises at least one lower energy rotational position and at least one higher energy rotational position. For example, the configuration illustrated in FIGS. **1A** and **1B** is the lower energy rotational position. For each 360° rotational cycle, the outer ring **110** has two lower energy rotational positions, or potential energy valleys, about 180° apart in which the major axis is generally parallel with the longitudinal axis thereof, and two higher energy rotational positions or potential energy peaks about 180° apart in which the major axis is generally parallel with the radial axis thereof. Consequently, the higher energy and lower energy rotational positions are about 90° apart. The outer ring **110** may have a potential energy profile that is generally sinusoidal, while the potential energy profile of the outer rings **110** may have a different shape, for example, generally saw-tooth, step-function, combinations thereof, or another suitable profile. Outer rings **110** with different cross-sectional shapes will have different potential energy profiles.

A consequence of the potential energy profile discussed above is referred to as "snap action" in the annular rotation of the outer ring **110.** Absent any applied rotational force, the outer ring **110** adopts a low energy geometry or potential energy valley as an equilibrium or detent position. Applying an annular torque to the outer ring **110** rotates or rolls the outer ring **110** around the annular axis, thereby increasing the potential energy of the outer ring **110,** until the outer ring **110** reaches the higher energy rotational position and potential energy peak. As the outer ring **110** passes over the potential energy peak, the stored potential energy therein is released as the outer ring **110** "snaps-to" or adopts the lower energy rotational position in falling into the next potential energy valley. Consequently, the outer ring **110** resists rotation out of the low energy rotational positions and snaps into the low energy rotational or detent positions when perturbed therefrom.

In a first direction of rotation referred to as "inversion" or "rolling in", the top of the outer ring **110** passes downwardly through the opening thereof. In a second direction of rotation referred to as "eversion" or "rolling out", the bottom of the outer ring **110** passes upwardly through the opening thereof. The potential energy profile may be generally symmetrical with respect to the direction of rotation, or not symmetrical, for example, steeper from valley to peak when rotating in one direction than when rotating in the opposite direction. For example, inversion may require a greater force than eversion. Some outer rings **110** with unsymmetrical potential energy profile have unsymmetrical cross sections.

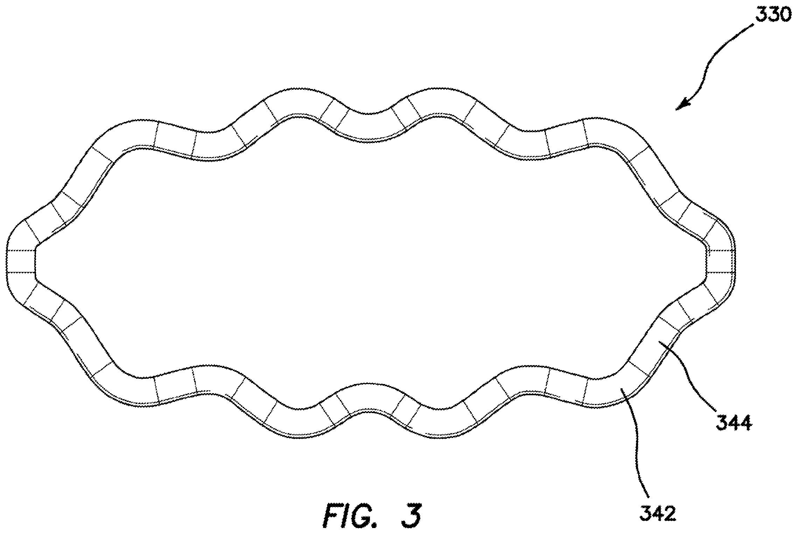

Returning to FIG. **1A****,** the inner ring **130** is deformable, comprising a flexible material, for example, a polymer for example, a flexible engineering plastic, the polymer may be an elastomer, for example, a thermoplastic elastomer. Alternatively, the inner ring 130 may be reshapeable, for example, comprising a plastically deformable or malleable elements, for example, metal and/or shape memory wires, strips, mesh, and the like. The deformable elements can be pleated or folded, for example, in an accordion fold or a fan fold. Alternatively, the reshapeable inner ring **130** may comprise clay, powders, granules, beads, and the like disposed in a covering or envelope. The reshapeable inner ring **130 may** comprise linked elements-for example, a link chain, a ball- and-socket chain, or a roller chain-covered, for example, with a flexible polymer. FIG. **3** is a perspective view of a reshapeable inner ring **330** comprising linked, alternating arcuate members **342** and straight members **344,** disposed end-to-end, defining a closed loop. Each arcuate member **342** is rotatable relative to an adjacent straight member **344** around a local longitudinal axis, resulting in a reshapeable inner ring **330.** Different numbers of arcuate **342** and straight **344** members may be employed. The length of each of the arcuate **342** and straight **344** members may be independently selected. The angle subtended by each arcuate member **342** may be independently selected. Some may comprise fewer or no straight members **344.** A conformation of the inner ring **330** may be lockable, for example, by applying tension or compression to the inner ring **330.** Reshapability permits a user to conform the inner ring **330** to the anatomy of the patient when placing the inner ring **330.**

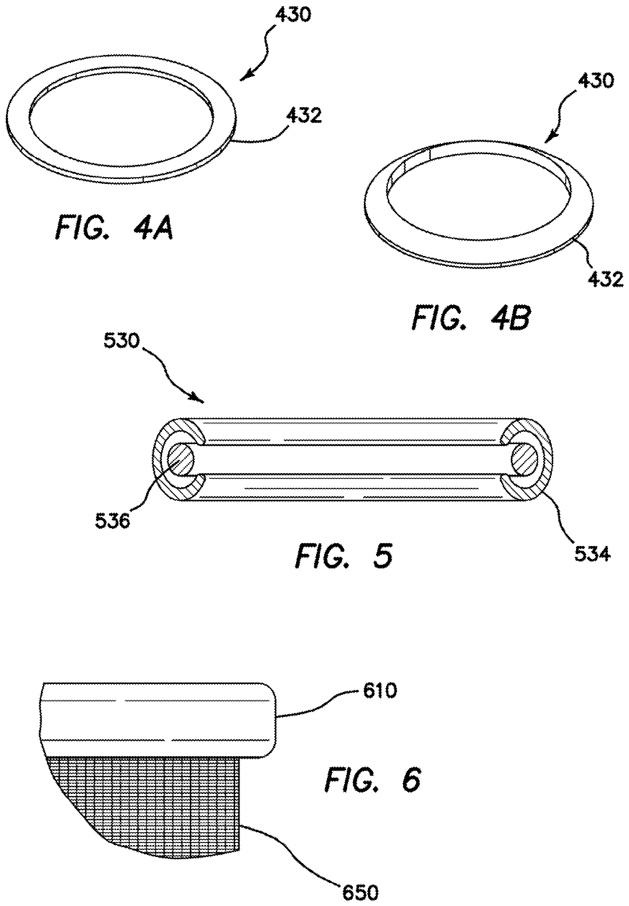

In FIG. **1A****,** a cross section of the inner ring **130** is generally circular. Alternatively, the inner ring **130** has another cross section, for example, oval, elliptical, flat, D-shaped, or any profile illustrated in FIGS. **2A-2K** for the outer ring **110.** The cross section of the inner ring may be thinned and/or flattened at least at the outer edge **432,** for example, a flat or thin wedge, resulting in an inner ring **430** with a washer-like shape as shown in FIGS. **4A** and **4B****.** The flattened outer edge permits a user to manipulate the edge into tight spaces when placing the inner ring **430,** for example, between muscle layers. The inner ring **130** may be molded and/or extruded as a single piece, or as a plurality of pieces that are assembled into the inner ring **130.**

The inner ring **130** may be collapsible and/or foldable, which facilitates inserting and/or removing the inner ring **130** through an incision or opening. For example, at least one notch, hinge, and/or weak point may be included, which facilitates folding thereof. The inner ring **130** may be arranged to disassemble, thereby permitting collapse of the inner ring **130.** For example, in some embodiments, the inner ring **130** comprises a member comprising two free ends that are brought together and coupled, thereby circularizing the inner ring **130.** In some of these arrangements, the coupled free ends are disassembled, thereby collapsing the inner ring **130.**The free ends may be coupled using a mechanical fastener, for example, at least one of a pin, a clip, a clasp, key, or the like. The fastener may comprise a breakable element, for example, a tab, that bridges the free ends. Disengaging or breaking the fastener uncouples the free ends.

In other arrangements, the inner ring **130** comprises an annular member coupled to a stiffening member. Disengaging and/or removing the stiffening member permits the annular member to collapse. For example, in, the stiffening member may comprise a ring-shaped portion around which the annular member is engaged. For example, in FIG. **5****,** the annular member **534** of the inner ring **530** comprises a C-shaped cross section with the opening of the C-shape generally facing the longitudinal axis of the annular member **534,** and at least a portion of the stiffening member **536** fits within the C-shape. In other arrangements, the opening of the C-shape faces another direction, for example, proximally, distally, or away from the longitudinal axis. Removing the stiffening member **536,** for example, by pulling on a tether secured thereto, as discussed below, permits collapsing the annular member **534.** In other arrangements, the stiffening member engages only a portion of the annular member. Again, removing the stiffening member permits collapsing the annular member.

In some arrangements, the inner ring **130** is inserted into a body cavity in the collapsed or folded state, then reconfigured into the retracting or deployed state therein.

The inner ring **130** may comprise a tether secured thereto. The tether facilitates removal of the inner ring **130,** for example, by pulling. In some embodiments, the tether facilitates folding or collapsing the inner ring. For example, pulling the tether may draw together portions of the inner ring **130** on either side of a notch, hinge, or weak point thereof, thereby folding the inner ring **130** and facilitating removal thereof. In some arrangements, the tether is secured to a mechanical fastener coupling the free ends of the inner ring **130** together. For example, the tether may removes or pulls free a pin, clip, or clasp, thereby unsecuring the free ends from each other. In some arrangements, the tether is coupled to a break-away element bridging the free ends of the inner ring **130** and breaks the break-away element when pulled, thereby unsecuring the free ends. In some arrangements, the tether is secured to a stiffening member of the inner ring **130,** and pulling the tether disengages the stiffening member from the annular member, thereby permitting the annular member to collapse.

In FIGS. **1A** and **1B****,** the sheath **150** is generally a cylindrical tube with a diameter substantially equal to inside diameters of the outer ring **110** and the inner ring **130.** However, the sheath **150** need not be cylindrical, for example it may be frustoconical, hourglass-shaped, D-shaped, oval, combinations, and the like. The sheath **150** may be fabricated as a seamless tube, or the sheath **150** may comprise at least one seam or a longitudinal pleats. In some embodiments, the sheath **150** comprises at least one longitudinal slit. In some embodiments, the sheath **150** comprises a plurality of bands, strips, and/or sheets extending between the outer ring **110** and the inner ring 130. The bands, strips, and/or sheets extend longitudinally and/or at a bias. In some embodiments, edges of adjacent bands, strips, and/or sheets overlap, thereby defining a tubular structure. In some embodiments, the sheath **150** comprises both a tubular component as well as at least one band, strip, and/or sheet. In some of these embodiments, at least some of the edges of adjacent bands, strips, and/or sheets do not overlap. A first end of the sheath **150** is coupled to the outer ring **110** and a second end of the sheath **150** is coupled to the inner ring **110.**

The flexible sheath **150** comprises an abrasion and/or puncture resistant material. The abrasion and/or puncture resistance of the sheath **150** improves the performance and reliability of the retractor **100** in procedures using sharp and/or pointed instruments, and/or prosthetic device, for example, in orthopedic procedures including hip procedures, hip replacement, and spinal procedures. Some of these procedure use instruments such as chisels, drills, rasps, scalpels, and the like. Embodiments of the retractor **100** are also useful in other types of procedures, for example, arthroscopic surgery, and even abdominal surgery. Embodiments of the abrasion and/or puncture resistant the sheath **150** protect the incision and/or opening in the body wall and surrounding tissue from damage from the instruments used in the surgical procedure. Some embodiments of the sheath **150** also reduce contamination in the surgical site, for example, from external bacteria, from tissues removed from the patient's body, and from surgical instruments and supplies.

The sheath material may have a puncture resistance of at least about 16 N (3.6 lb) under FED-STD-101/2065 (Puncture Resistance and Elongation Test), or a puncture resistance of at least about 20 N (4.5 lb), at least about 30 N (6.7 lb), at least about 40 N (9 lb), at least about 50 N (11 lb), at least about 60 N (13.5 lb), or at least about 100 N (22.5 lb).

### EXAMPLE 1

Puncture resistance was measured according to FED-STD-101/2065 for a polyurethane laminated fabric (PUL-2mil, Seabright) comprising a polyester knit fabric and a 50 µm (2 mil) polyurethane layer laminated to one face of the fabric. In the test, a 3.175 mm (0.125 in) rounded shaft was contacted with a 7.6 cm × 7.6 cm (3 in × 3 in) fabric sample at 7.6 cm/min (3 in/min). The force at penetration is the penetration resistance. The test was performed on 15 samples with the rounded shaft contacting the polyester face of the fabric with an average puncture resistance of 61.07 N (13.73 lb). The average puncture resistance for 15 tests on the polyurethane side was 53.56 N (12.04 lb).

### EXAMPLE 2

Puncture resistance of a 76 µm (4 mil) polyether polyurethane film (PELLETHANE^{®} 2363, Lubrizol) used in current retractor sheaths was measured as above. The average for 15 tests was 12.46 N (2.80 lb).

Embodiments of the sheath comprise sheets, membranes, fibers, and/or strands of one or more materials that endow the sheath with the abrasion and puncture resistance. Suitable sheets, membranes, fibers, and/or strands comprise at least one of natural polymers, semi-synthetic polymers, synthetic polymers, metal, ceramic, glass, carbon fiber, carbon nanotubes, and the like. Suitable natural polymers include cellulose, silk, and the like. Semi-synthetic fibers include nitrocellulose, cellulose acetate, rayon, and the like. Suitable synthetic fibers include polyester, aromatic polyester, polyamide (NYLON^{®}, DACRON^{®}), aramid (KEVLAR^{®}), polyimide, polyolefin, polyethylene (SPECTRA^{®}), polyurethane, polyurea, polyvinyl chloride (PVC), polyvinylidene chloride, polyether amide (PEBAX^{®}), polyether urethane (PELLETHANE^{®}), polyacrylate, polyacrylonitrile, acrylic, polyphenylene sulfide (PPS), polylactic acid (PLA), poly(diimidazopyridinylene-dihydroxyphenylene) (M-5); poly(p-phenylene-2,6-benzobisoxazole) (ZYLON^{®}), liquid crystal polymer fiber (VECTRANO), and the like, and blends, copolymers, composites, and mixtures thereof. Suitable metals include stainless steel, spring steel, nitinol, super elastic materials, amorphous metal alloys, and the like.

FIG. **6** is a detailed view of a portion of the outer ring **610** and sheath **650,** which comprises a fabric or textile. In some embodiments, the fabric or textile comprises, for example, at least one of a woven fabric, a non-woven fabric, a knit fabric, a double-knit fabric, a mesh, a braided fabric, and a braided mesh fabric. Suitable fabrics comprise monofilament fibers and/or yarns. Other suitable fabrics comprise twisted and/or braided yarns. Suitable yarn materials are described in the previous paragraph. Some embodiments of the fabric comprise a combination of fibers, for example, different warp and weft yarns in woven or mesh fabrics, or a combination of yarns in knit or braided fabrics. Some embodiments of the fabric are substantially nondistensible, while other embodiments are distensible. In some embodiments, the fabric resists tear propagation in the event of damage thereto, for example, from inadvertent puncturing or cutting by a surgical instrument, or from purposeful puncturing in securing the sheath **150** as described below. Examples of such fabrics include rip-stop fabrics, certain knits, double knits, and braided mesh fabrics. In some embodiments, the orientation of fabric reduces the likelihood of snagging or otherwise obstructing an instrument as it is inserted through the sheath. For example, in some embodiments, a smoother surface of the fabric faces a longitudinal axis or inside of the sheath. In some embodiments, the fabric is oriented on a bias, or with ridges or troughs generally parallel with the longitudinal axis of the sheath. Examples of suitable fabrics include rip-stop polyamide (Nylon^{®}), Oxford weave fabrics, abrasion-resistant polyester and/or polyamide fabrics (Cordura^{®}), braided monofilament fabrics, and the like.

Some embodiments of the sheath material comprises a composite comprising a fabric or textile, for example, at least one of a coated fabric, a laminated fabric, and a fabric embedded in a polymer. Coatings and/or laminations are disposed on one face or both faces of the fabric. Suitable coatings and laminating materials include polymers, for example, at least one of polyurethane, polyether, PVC, polyvinylidene chloride, silicone, styrenebutadiene, polyethylene, polypropylene, ethylene-propylene copolymer, polyisoprene, ethylene vinyl acetate (EVA), ethylene-propylene-diene monomer (EPDM), polyamide (MYLAR^{®}), polyether block amide (PEBAX^{®}), polyether urethane (PELLETHANE^{®}), composites, blends, mixtures, and the like. An example of a suitable composite fabric is polyurethane laminated fabric (PUL). Some embodiments of the coating or lamination modify gas and/or moisture permeability through the sheath material, for example, by controlling the size of pores therethrough. For example, decreasing moisture permeability reduces dehydration of the retracted tissue and/or creates a barrier to pathogens such as bacteria. Increasing gas and moisture permeability permits hydrating and/or oxygenating the retracted tissue. Some materials are selectively permeable to certain fluids. For example, some embodiments of PVC are oxygen permeable and moisture impermeable, thereby permitting simultaneously oxygenating tissue while reducing dehydration. Some embodiments of the coating or lamination comprise an antibacterial or antimicrobial agent. In some embodiments, the antibacterial or antimicrobial agent is a surface agent or is integral to the material. Examples of suitable antibacterial or antimicrobial agents include iodine, antibiotics, silver, triclosan, biocides, and the like. Some embodiments of the coating or lamination provide a smoother and/or lower friction inside surface, which reduces the likelihood of instrument damage to the sheath **150.**

Some embodiments of the sheath **150** comprise a composite comprising a fiber-reinforced polymer film or membrane. Suitable fibers or strands are discussed above. Suitable polymer film materials include at least one of materials discussed above as coating and laminating materials. In some embodiments, the fibers are sandwiched between polymer film layers. In some embodiments, the polymer film layers are independently selected. For example, in some embodiments, the outer layer provides desirable tissue contact properties discussed above, while the inner layer is puncture resistant.

Some embodiments of the sheath **150** comprise a plurality of layers, for example, a fabric layer and a polymer film layer, or a fabric layer sandwiched between polymer film layers. In some embodiments, the layers are secured to each other. In other embodiments, the layers are independent of, or not secured to each other, for example, a polymer film layer and a layer comprising a plurality of strips or bands as discussed above.

Some embodiments of the sheath **150** comprise a fluid-permeable layer disposed on a fluid-impermeable layer, with the fluid-impermeable layer disposed on the inside of the sheath **150.** The fluid-permeable layer contacts the wound margins, thereby permitting a user to supply pressurized fluid and/or apply vacuum to the wound margins. For example, in some embodiments, oxygen, moisture, therapeutic agent, and/or other fluids are supplied to the wound margins. In some embodiments, applying vacuum promotes bleeding, thereby reducing tissue necrosis. Embodiments of the fluid-permeable layer comprise at least one of open cell foam, fabrics, non-woven fabrics, and knit fabrics.

In other embodiments, the sheath **150** is stretchable longitudinally. In some embodiments, longitudinal and circumferential stretch characteristics of the sheath **150** are the same, that is, the stretch is isotropic. In other embodiments, longitudinal and circumferential stretch characteristics of the sheath **150** are different, that is, the stretch is anisotropic. For example, in some embodiments, the sheath **150** has greater circumferential stretch than longitudinal stretch.

In other embodiments, the sheath **150** has substantially no or little longitudinal stretch, that is, is non-distensible longitudinally. Consequently, a retraction force exerted on an incision or opening by the sheath **150** remains substantially constant over the course of a procedure. In some embodiments, the sheath **150** is radially or circumferentially expandable. For example, some embodiments of a tubular sheath **150** comprise a woven material, as discussed below, that is expandable or stretchable circumferentially, that is, perpendicular to the longitudinal axis. Some embodiments comprise an elastomeric membrane or film, and longitudinal non-stretchable elements. For example, some embodiments of the sheath **150** comprise a composite comprising an elastomeric film and longitudinally disposed, non-stretchable fibers, as discussed above. The fibers make the sheath **150** longitudinally non-stretchable, while the polymer film permits radial expansion. Embodiments of the sheath **150** comprising non-stretchable longitudinal strips and an elastomeric membrane are also longitudinally non-stretchable and radially expandable. Embodiments of a sheath **150** comprising a non-stretchable tube comprising one or more longitudinal slits and/or pleats are longitudinally non-stretchable and radially expandable. Embodiments of a sheath **150** comprising a plurality of non-stretchable longitudinal strips or bands are also longitudinally non-stretchable and radially expandable.

In some embodiments, at least a portion of the sheath **150** is transparent or transparent, thereby providing a view of the retracted tissue. In some embodiments comprising a polymer membrane or film, the polymer membrane or film is transparent or transparent.

In some embodiments, the sheath **150** comprises a proximal portion **152** with different properties than a distal portion **154** thereof. For example, in some embodiments, the proximal portion **152** has greater flexibility than the distal portion **154,** thereby facilitating winding or rolling the sheath **150** around the outer ring **110.** In other embodiments, the proximal portion **152** comprises one of a hook and a loop of a hook-and-loop fastener, thereby providing adjustability in embodiments using hook-and-loop fasteners, discussed below. Some embodiments of the sheath **150** further comprise a middle portion **156** disposed between the proximal **152** and distal **154** portions. For example, some embodiments of the proximal portion **152** and the distal portion **154** of the sheath comprise a tear-resistant material for use with outer rings **110** and inner rings **130** comprising teeth, as described below. In some embodiments, the proximal portion **152** and the distal portion **154** of the sheath comprise an elastomeric material, and the middle portion comprises a longitudinally non-distensible material, as described above.

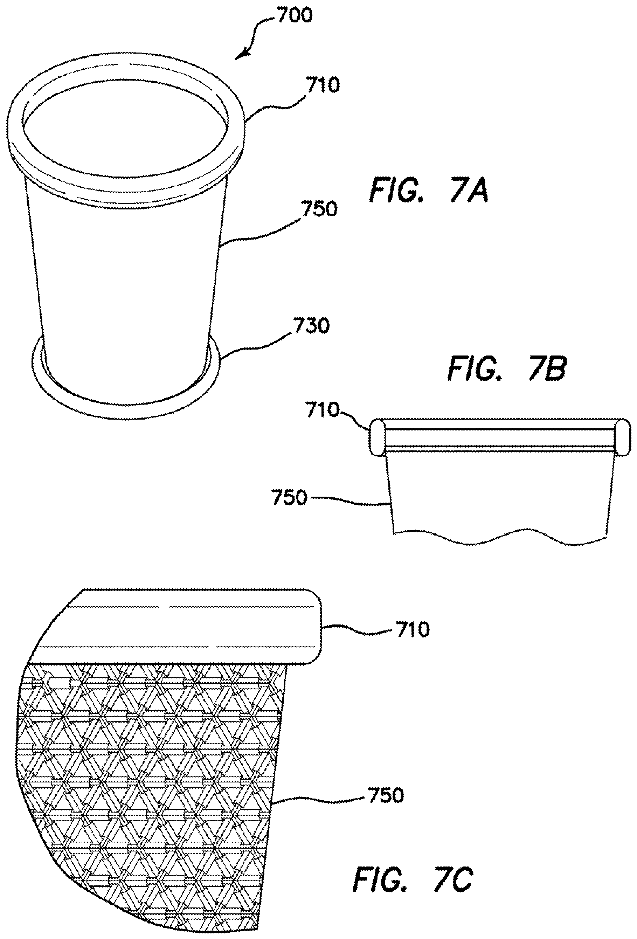

FIG. **7A** is a perspective view and FIG. **7B** is a side cross section of another embodiment of a wound retractor **700** that is generally similar to the embodiment described above. The retractor **700** comprises an outer ring **710,** an inner ring **730,** and a sheath **750.** In the illustrated embodiment, the outer ring **710** has a larger diameter than the inner ring **730.** Consequently, the sheath **750** is generally frustoconical or funnel-shaped, tapering or converging from a proximal end, coupled to the outer ring **710,** to a distal end, coupled to the inner ring **730.** Also, the outer ring **710** has a generally oval shape rather than the figure-8 shape of the embodiment illustrated in FIGS. **1A** and **1B****.** As shown in a detailed view of the outer ring **710** and sheath **750** in FIG. **7C****,** the sheath **750** comprises a knit fabric in the illustrated embodiment.

The embodiment of the retractor **700** is useful in procedures in which the inner ring **730** is inserted through a smaller body opening. The larger outer ring **710** improves protection of the body opening and surrounding tissue. Examples of such procedures include orthopedic hip replacement, vaginal retraction, and rectal retraction.

Other embodiments of the retractor comprise at least one of an outer anchor member and an inner anchor member different from the embodiments described above.

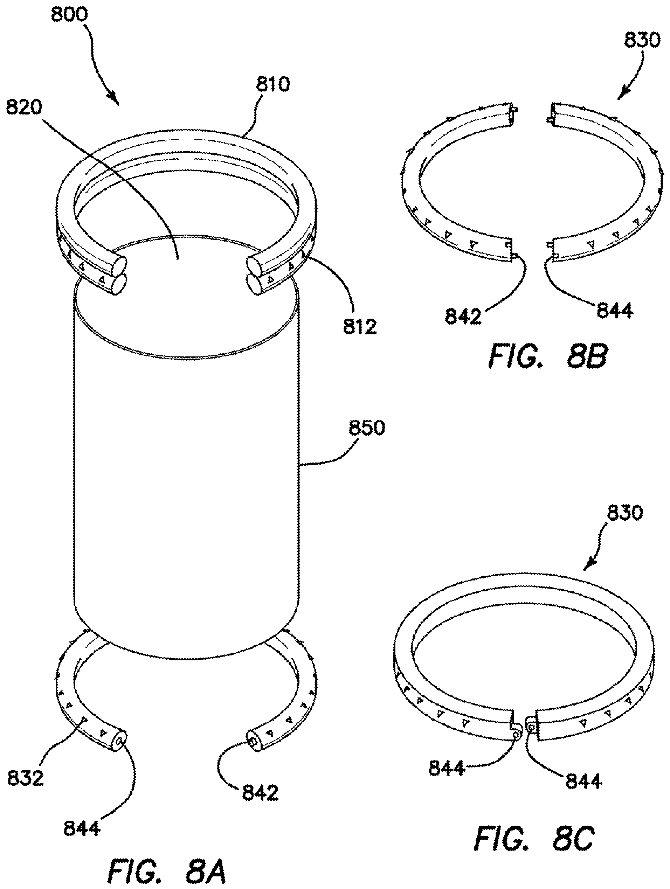

FIG. **8A** illustrates a perspective view of another embodiment of a retractor **800** generally similar to the embodiments described above. In the illustrated embodiment, an outer ring **810,** inner ring **830,** and sheath **850** are unassembled. The illustrated retractor **800** is provided as separate components, which are assembled by the user. For example, in some embodiments, individual components are selected from a kit according to the requirements of a particular procedure. For example, embodiments of the kit comprise at least one of outer rings **810** with different diameters, inner rings **830** with different diameters, sheaths **850** with different diameters, sheaths **850** with different lengths, sheaths comprising different materials, and the like. In some embodiments, the retractor **800** is disassemblable, and one or more of the outer ring **810,** the inner ring **830,** and the sheath **850** is reusable, for example, autoclavable.

The outer ring **810** is illustrated in a non-circularized configuration. In the illustrated embodiment, ends of the non-circularized outer ring **810** are coupled using a coupler **820.** The outer ring **810** comprises a plurality of fasteners **812,** which secure the sheath **850** to the outer ring **810.** The inner ring **830** is also in a non-circularized configuration, and comprises a circumferentially facing pin or peg **842** on a first end thereof, and a corresponding circumferentially facing opening **844** disposed on a second end. The inner ring **830** also comprises a plurality of fasteners **832,** which secure the sheath **850** to the inner ring **830.** In the illustrated embodiment, the fasteners **812** and **832** comprise hooks, which puncture the sheath **850,** thereby securing the sheath to the outer ring **810** and inner ring **830,** respectively. In some embodiments, the fasteners **812** and/or **833** are bendable, which permits a user to further secure the sheath **850.** In other embodiments, each of the outer ring **810** and inner ring **830** independently comprises fasteners for the sheath **850,** for example, hooks, clips, clamps, pins, wires, hook-and-loop fasteners, laces and eyelets, and the like. For example, in some embodiments, the fastener comprises a wire that passes through eyelets disposed on both the sheath **850,** and the outer ring **810** or the inner ring **830.** In other embodiments, the outer ring **810** and/or inner ring **830** comprises two interlocking rings that capture the sheath **850** therebetween, thereby securing the sheath thereto. In some embodiments, the interlocking rings snap together, screw together, clip together, and the like.

In the illustrated embodiment, the inner ring **830** comprises a pin-and-hole **842** and **844** system that couples together the free ends thereof. FIG. **8B** is a perspective view of an inner ring **830** comprising another embodiment of a pin-and-hole system comprising a plurality of sections, each comprising pins **842** disposed on both ends of the inner ring **830** and corresponding, mating holes **844** disposed on both ends of the inner ring **830,** which permit a user to couple the free ends of the inner ring **830.**

FIG. **8C** is a perspective view of another embodiment of an inner ring **830** comprising a pin-and-hole system comprising a radial hole **844** disposed on each free end of the outer ring **830,** which together with a pin (not illustrated), couple the free ends of the inner ring **830.** As discussed above, some embodiments of the retractor **800** comprise a tether suitable for pulling a pin free, thereby collapsing the inner ring **800.**

Those skilled in the art will understand that similar arrangements for circularizing the outer ring **810** and the inner ring **830** described above conjunction with the embodiments illustrated in FIGS. **8A-8C** are also applicable to the inner ring **830** and the outer ring **810,** respectively. In the embodiment illustrated in FIG. **8A****,** the outer ring **810** is rotatable around an annular axis thereof. In other embodiments, the outer ring is not rotatable around an annular axis. In some of these embodiments, the sheath **850** is not tensioned by wrapping around the outer ring **810.** Instead, the sheath **850** is threaded through a portion of the access channel extending through the center of the outer ring **850,** then tensioned by pulling the sheath **850** distally from the inner ring **830.** The tension is maintained by engaging the sheath **850** to the fasteners **812,** hooks in the illustrated embodiment.

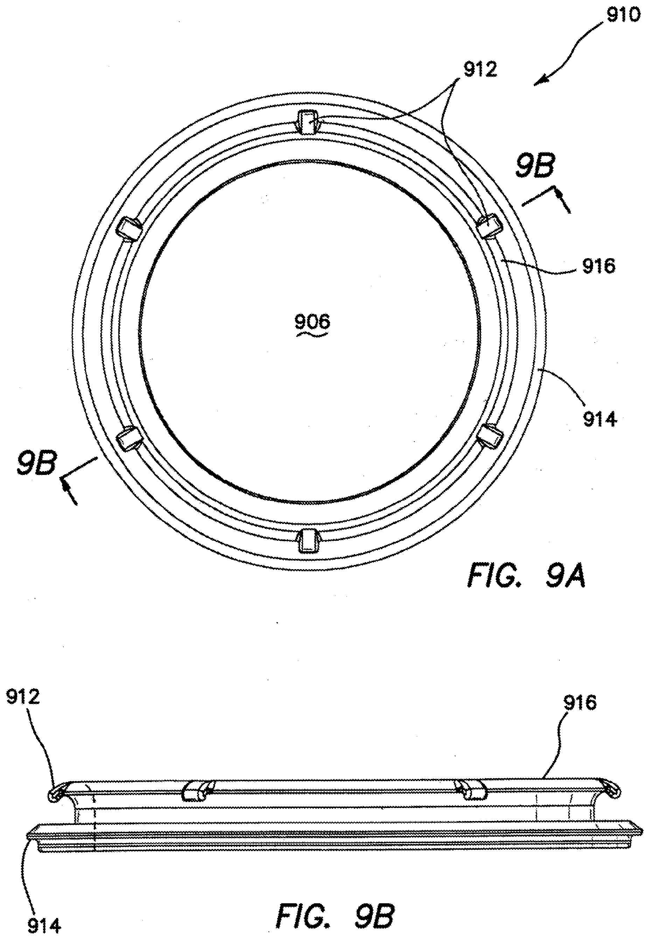

FIG. **9A** is a top view and FIG. **9B** is a side view of another embodiment of an outer ring **910** comprising a lower flange **914,** a concentric upper flange **916,** and a plurality of fasteners or hooks **912** extending radially outwards and distally from the upper flange **916.** The outer ring **910** is rigid or semi-rigid and is not rotatable around an annular axis. In use, a proximal end of the sheath is threaded proximally out through a portion of an access channel **906** extending through the outer ring **910,** pulled proximally, thereby tensioning the sheath, and the sheath engaged to the hooks **912,** thereby maintaining a desired tension on the sheath. In the illustrated embodiment, the hooks **912** are blunt and do not penetrate the sheath. In other embodiments, the hooks **912** are pointed and penetrate the sheath.

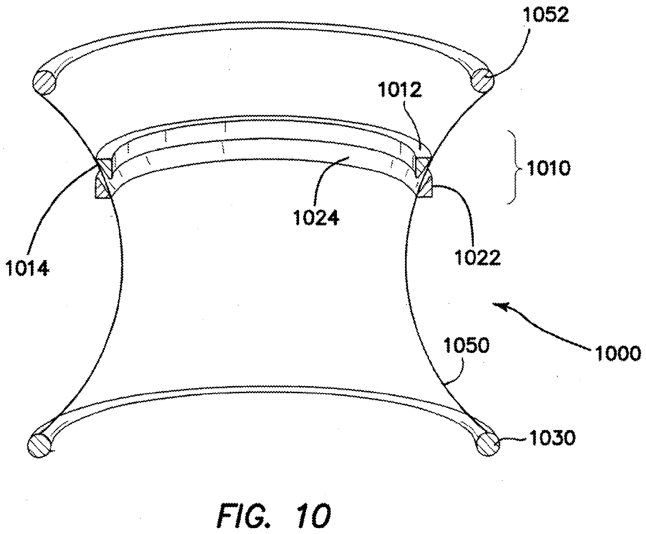

FIG. **10** is a side partial cross section of another embodiment of a retractor **1000,** similar to the embodiments described above, comprising an outer anchor **1010,** an inner anchor **1030** and a sheath **1050.** In the illustrated embodiment, the outer anchor **1010** comprises a proximal ring **1012** and a distal ring **1022,** which nest together. The nesting surfaces **1014** and **1024,** respectively, are frustoconical or wedge-shaped, with a distal diameter smaller than a proximal diameter. In some embodiments, at least a portion of the nesting surfaces **1014** and **1024** comprise steps. With the sheath **1050** disposed between the proximal ring **1012** and the distal ring **1014** as shown in FIG. **10****,** pulling the sheath **1050** distally, for example, when the sheath **1050** is under tension while retracting tissue, draws the proximal ring **1012** distally, thereby seating the nesting surface **1014** of proximal ring **1012** against the nesting surface **1024** of the distal ring **1024.** This wedging action locks the sheath **1050** between the proximal ring **1012** and the distal ring **1022,** thereby resisting further distal movement of the sheath. In contrast, the sheath **1050** is freely movable proximally because the sheath motion unseats the proximal ring **1012** from the distal ring **1024.** In the illustrated embodiment, a gripping element **1052** is disposed at a proximal end of the sheath **1050,** which improves a user's grip when applying traction or tension to the sheath **1050.**

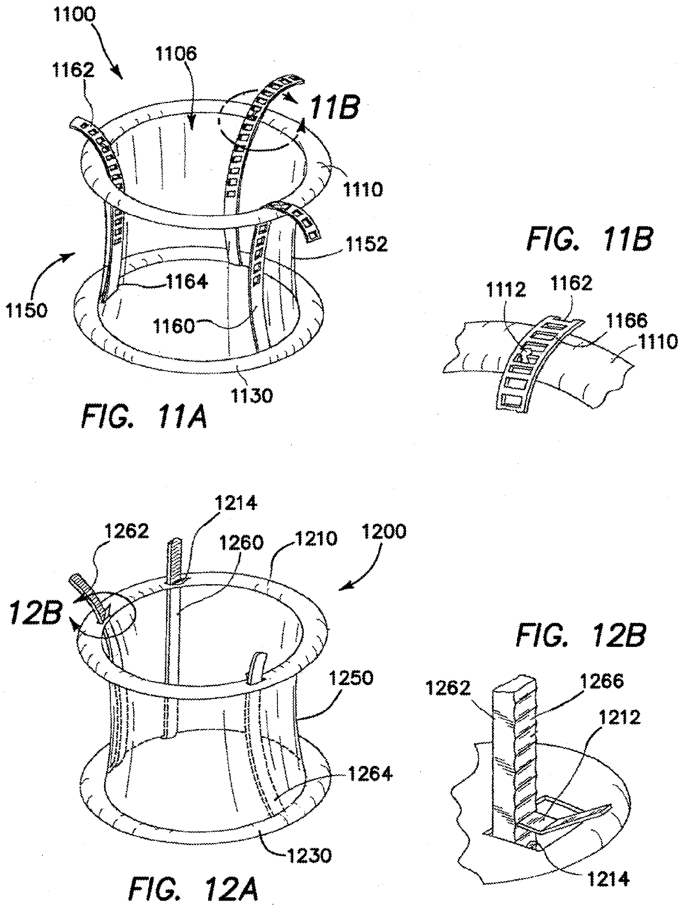

FIG. **11A** is a perspective view of another embodiment of a retractor **1100,** generally similar to the embodiments described above, comprising an outer ring **1110,** an inner ring **1130,** and a sheath **1150.** In the illustrated embodiment, the sheath **1150** comprises a tubular membrane **1152** extending between the outer ring **1110** and the inner ring **1130,** and a plurality of elongate bands **1160,** each comprising a proximal end **1162** and a distal end **1164.** The distal end **1162** of the band **1160** is secured to the distal ring **1130.** The proximal end **1162** comprises a ladder-like section comprising a plurality of rungs defining opening **1166** (FIG. **11B**) therebetween. The proximal ends **1162** extend through an access channel **1106** and through the outer ring **1110.** As shown in FIG. **11B****,** which is a detail view of the outer ring **1110** and proximal end **1162** of a band **1160,** the outer ring **1110** further comprises a plurality of fasteners or hooks **1112** dimensioned to engage the openings **1166** in the proximal end **1162** of the band **1160,** thereby maintaining a desired tension or retraction force between the outer ring **1110** and inner ring **1130.** Some embodiments of the outer ring **1110** comprise a greater number of hooks **1112** than the number of bands **1160,** which provides greater flexibility in engaging each band **1160** to the outer ring **1110.**

FIG. **12A** is a perspective view of an embodiment of a retractor **1200** generally similar to the embodiments described above, and in particular, to the embodiment illustrated in FIGS. **11A** and **11B****.** The retractor **1200** comprises an outer ring **1210,** an inner ring **1230,** and a sheath **1250,** which in the illustrated embodiment, comprises a flexible membrane **1252** and a plurality of proximally extending bands **1260.** A distal end **1264** of each band **1260** is secured to the distal ring **1230.** A proximal end **1262** of each band **1260** extends through an opening **1214** through the outer ring **1210.** As best seen in the detail view in FIG. **12B****,** the proximal end **1262** of the band **1260** comprises a plurality of transverse grooves **1266** which define a ratcheting surface. The outer ring **1210** comprises a pawl **1212** juxtaposed with the opening **1214.** The pawl **1212** engages the grooves **1266** of the ratcheting surface. The illustrated embodiment of the pawl **1212** is also disengageable from the grooves **1266.** Embodiments of the ratcheting surface and pawl **1212** are similar to corresponding elements in cable ties and zip ties. The grooves **1266** and pawl **1212** in the engaged position maintain a desired position of the band **1260,** and consequently, the relative positions of the outer ring **1210** and the inner ring **1230.** Hence, the mechanism permits a user to adjust and maintain the relative positions of the outer ring **1210** and the inner ring **1230,** and consequently, a desired tension in the bands **1260** in retracting tissue.

Those skilled in the art will understand that similar principles are applicable to similar embodiments, for example, in which the bands comprise a plurality of enlarged or bead-like portions that engage suitably dimensioned notches in an outer ring, or in which bands and the outer ring comprise complementary hook-and-loop fasteners. In other embodiments, the bands are laces that alternately pass through openings in the outer ring and inner ring and are lockable, for example, by tying together, tying off, clamps, clips, wedges, and the like.

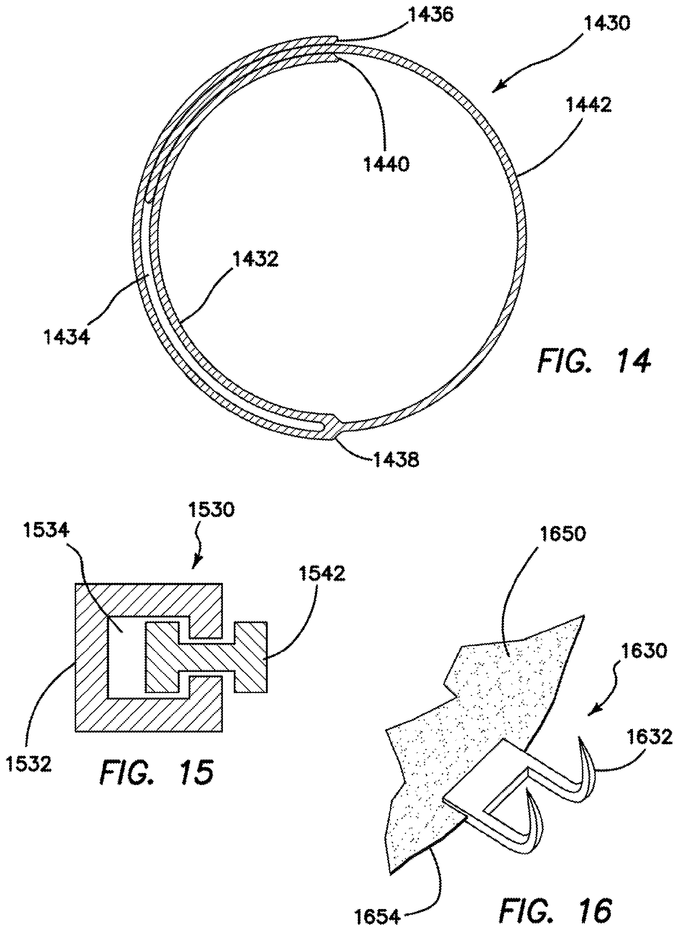

In an embodiment illustrated in a top cross section in FIG. **14****,** the inner ring **1430** has an adjustable diameter. In the illustrated embodiment, the inner ring **1430** comprises an elongate, tubular body **1432** defining a lumen **1434,** wherein the body **1432** comprises a first end **1436,** a second end **1438,** and an opening **1440** into the lumen **1434** at the first end **1436.** An elongate shaft **1442** extends from the second end **1438** of the body **1432.** In the illustrated embodiment, cross sections of the lumen **1434** and opening **1440** have the same dimensions. The shaft **1442** is dimensioned to be received through the opening **1440** and into the lumen **1434,** thereby defining a ring. Telescoping the shaft **1442** in or out of the body **1432** adjusts the diameter of the inner ring **1430.**

In the embodiment of the inner ring **1530** illustrated in cross section in FIG. **15****,** the body **1432** is C-shaped, defining a channel **1534** into which a suitably dimensioned shaft **1542** is received.

In some embodiments of the above inner rings, the shaft is selectively lockable in the body, for example, using a ratchet and pawl, compressing the opening and/or lumen/channel, threads, locknuts, lock rings, friction, and the like. In the embodiment illustrated in FIG. **14****,** a top view of the inner ring **1430** is generally circular. In other embodiments, the inner ring has another shape as described above. Other embodiments comprise a plurality of bodies and shafts. In some embodiments, the body is two-ended, that is, each end of the body is dimensioned to receive a shaft telescopically, and the shaft is also two-ended, that is, each end of the shaft is insertable into a body. Some embodiments of the outer ring are similarly adjustable.

In an embodiment illustrated in perspective in FIG. **16****,** the inner anchor **1630** comprises a plurality of hooks **1632** disposed around a distal end **1654** of the sheath **1650,** which when inserted into tissue, anchor the distal end **1654** of the sheath **1650.** In the illustrated embodiment, two hooks **1632** are combined into a single anchoring unit. Other embodiments use individual hooks **1632** in each anchoring unit, multiple hooks **1632,** or a combination thereof. Embodiments of outer anchors also comprise similar hooks.

Some embodiments of the outer anchor comprise an adhesive. In these embodiments a proximal portion **152** of the sheath **150** (FIG. **1A**) is simply adhered to a patient's skin, for example, using one at least one of a pressure sensitive adhesive, a UV curing adhesive, a two-part adhesive, and the like.

FIGS. **17A-17C** illustrate embodiments of retractors **1700** similar to the embodiments discussed above, with those of FIGs 17A and 17C being in accordance with the present invention, comprising an outer ring **1710,** and inner ring **1730,** and a sheath **1750.** In the illustrated embodiments, the sheath **750** comprises metal fibers and/or strands, for example, stainless steel, nitinol, titanium, and the like, which are autoclavable. The embodiment of the sheath **1750** illustrated in FIG. **17A** comprises a mesh comprising linked loops **1752,** for example, similar to chain mail. In other embodiments, the sheath **1750** comprises loops **1752** that are not interlinked, but are joined, for example, with thread or wire extending through adjacent loops longitudinally, circumferentially, diagonally, or a combination thereof. In the embodiment of the retractor **1700** illustrated in FIG. **17B****,** the sheath **1750** comprises braided wire. In the embodiment of the retractor **1700** illustrated in FIG. **17C****,** the sheath **1750** comprises a plurality of chains **1752,** which are an embodiment of the bands, strips, and/or sheets discussed above. In the illustrated embodiment, the outer ring **1710** is similar to the embodiments illustrated in FIGS. **8A****,** **9A****,** and **11A****.** In some embodiments, the sheath **1750** further comprises a polymer film is disposed around the metal components in use, thereby protecting the incision or wound, as discussed above. In some embodiments of the sheath **1750** or portion thereof illustrated in FIGS. **17A-17C****,** the metal component is supplemented by another material, for example, an engineering plastic, ceramic, a fiber reinforced composite, and the like.

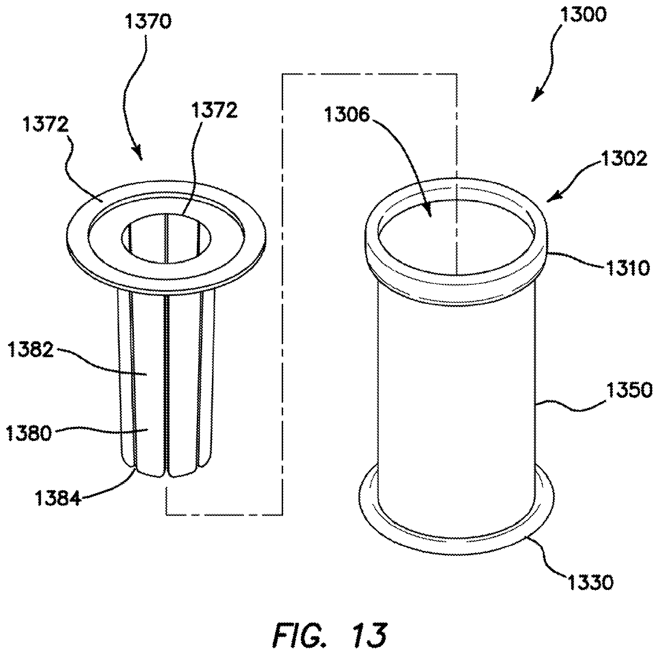

FIG. **13** is an exploded view of an embodiment of a retractor **1300,** similar to the embodiments described above, comprising an outer ring **1310,** an inner ring **1330,** and a tubular sheath **1350,** and further comprising a shield **1370.** The shield **1370** is dimensioned for insertion into an access channel **1306.** The shield comprises a proximal radial flange **1372** and a tubular portion **1380** extending distally from an opening **1374** in the flange **1372.** In the illustrated embodiment, the tubular portion **1380** comprises a plurality of elongate fingers **1382,** which define narrow gaps **1384** therebetween. In other embodiments, the tubular portion has a different configuration, for example, overlapping fingers, a tube, and the like. In the illustrated embodiment, the fingers **1382** converge. In other embodiments, the fingers do not converge, for example, are generally parallel, or diverge. In some embodiments, distal ends of the fingers **1382** diverge, thereby defining a funnel that directs instruments on withdrawal.

The flange **1372** is dimensioned to be supported either by the outer ring **1302,** or in the illustrated embodiment, by tissue (skin) around an incision or opening. The opening **1374** is dimensioned to receive the largest instrument contemplated in a procedure. The flange **1372** also a portion of the sheath **1350** on which it is disposed and the underlying tissue. In the illustrated embodiment, the flange **1372** also defines a funnel for instrument insertion into the tubular portion **1380.**

The shield **1370** is manufactured as a single assembly or as multiple components that are assembled into the final product. The illustrated embodiment of the shield **1370** comprises flexible or semi-rigid fingers **1382.** The flange **1372** is rigid, semi-rigid, or flexible. The shield **1370** suitably comprises materials similar to those described above as suitable for the sheath. In some embodiments, the shield **1370** comprises a polymer. In some embodiments, the inner surfaces of the tubular portion 1380 are smooth.

In use, the retractor **1300** is used to retract an incision or opening as described below. The shield **1370** is then inserted into the access channel **1306** through the proximal end **1302** of the retractor **1300.** The shield **1370** provides additional protection to the sheath **1350,** and consequently, the retracted tissue. The shield **1370** may be removed where additional space is required for a procedure, or where the procedure presents reduced risk of tissue injury or trauma.

A method for retracting a body wall is described with reference to the retractor **100** illustrated in FIGS. **1A** and **1B****,** although the method is applicable to any of the retractors described herein.

The inner anchor or inner ring **130** is inserted though an incision, wound, or opening in the body wall. In some embodiments, inserting the inner ring **130** is facilitated by folding or collapsing the inner ring **130** prior to insertion. After insertion, the inner ring **130** is then unfolded, expanded, or deployed in the tissue in interior of the body, as described above. On completing this step, the inner ring **130** is disposed within the body, the sheath **150** extends out of the incision, and the outer anchor or outer ring **150** is disposed outside the body.

The distal end **152** of the sheath **150** is then pulled towards the user, thereby tensioning the sheath **150.** The outer anchor **110** is then deployed. In the illustrated embodiment, deploying the outer anchor comprises rotating the outer ring **110** around the annular axis, thereby rolling the sheath **150** therearound, and shortening the length of the sheath **150** between the inner ring **130** and the outer ring **110.** As discussed above, the outer ring **110** is rotatable in two directions: rolling-in or inversion, and rolling-out or eversion. Either rotational direction effectively rolls the sheath **150** therearound. As discussed above, in some embodiments, one direction is preferred over the other. On continued rolling, the outer ring **110** contacts the outer surface of the body wall, while the inner ring **130** contacts the inner surface of the body wall. Continued rolling of the outer ring **110** creates a desired tension on the sheath **150,** thereby retracting the incision. Rolling the outer ring **110** is discontinued at a desired degree of retraction.

Also as discussed above, rotating the outer ring **110** around the annular axis occurs in discrete steps or increments. In the illustrated embodiment, the outer ring **110** comprises equilibrium or detent positions 180° apart. In these equilibrium or detent positions, the outer ring **110** resists rotation around the annular axis. Consequently, the outer ring **110** resists unrolling under the retracting tension of the sheath **150.**

When unretracting or releasing the retractor, the sheath **150** is unrolled from the outer ring **110** by reversing the rolling direction of outer ring **110,** thereby releasing the tension in the sheath **150.** The inner ring **130** is then removed from the body cavity. As discussed above, in some embodiments, the inner ring **130** is folded or collapsed within the body cavity, thereby facilitating removal. As discussed above, in some embodiments, removing the inner ring **130** may comprise pulling a tether secured thereto.

While certain embodiments have been particularly shown and described with reference to exemplary embodiments thereof, the scope of the invention is defined by the following claims.

## Claims

1. A tissue retractor (1700) comprising:
a longitudinal axis defining an instrument access channel extending from a proximal end to a distal end;
an outer ring (1710);
an inner ring (1730); and
a flexible sheath (1750) extending between the outer ring (1710) and the inner ring (1730), wherein the instrument access channel extends through the outer ring (1710), the inner ring (1730), and the flexible sheath (1750), wherein the outer ring (1710) is configured to roll the flexible sheath (1750) around the outer ring (1710) to provide the flexible sheath (1750) with a radial retraction force sufficient to stretch an incision to a desired diameter, **characterized in that** the flexible sheath (1750) is a flexible metal sheath and comprises one of:
a mesh comprising a plurality of metal loops (1752) that are linked,
a plurality of metal loops (1752) that are not interlinked but are joined by a thread or wire extending through adjacent loops of the plurality of metal loops (1752), or
a plurality of metal chains.

2. The tissue retractor (1700) of claim 1, wherein the flexible sheath (1750) is puncture resistant.

3. The tissue retractor (1700) of claim 1, wherein the plurality of metal loops (1752) joined by the thread or wire or the plurality of metal chains are connected to the outer ring (1710) in that the outer ring (1710) may roll the plurality of metal loops (1752) or the plurality of metal chains around the outer ring (1710) as the outer ring (1710) rolls.

4. The tissue retractor (1700) of claim 1, wherein the flexible sheath (1750) is reusable.

5. The tissue retractor (1700) of claim 1, wherein the flexible sheath (1750) is detachably connected to the outer ring (1710) at the proximal end and the inner ring (1730) at the distal end.

6. The tissue retractor (1700) of claim 1, wherein the flexible sheath (1750) comprises stainless steel, nitinol, or titanium.

7. The tissue retractor (1700) of claim 1 further comprising a polymer film disposed around the flexible sheath (1750) and connected to the outer ring (1710) in which the outer ring (1710) may roll the flexible sheath (1750) and the polymer film around the outer ring (1710).

8. The tissue retractor (1700) of claim 1 wherein the flexible sheath (1750) further comprises one or more of a plastic, ceramic, or a fiber reinforced composite.

9. The tissue retractor (1700) of claim 1, wherein the flexible sheath (1750) is autoclavable.

10. A tissue retractor kit for the tissue retractor (1700) of claim 1, comprising:
the outer ring (1710) having a first plurality of fasteners (812) disposed around a circumference of the outer ring (1710);
the inner ring (1730) having a second plurality of fasteners (832) disposed around a circumference of the inner ring (1730); and
the flexible metal sheath (1750) having a distal end and a proximal end,
wherein the tissue retractor (1700) is configured to be assembled by securing the proximal end of the flexible metal sheath (1750) to the first plurality of fasteners (812) and the distal end of the flexible metal sheath (1750) to the second plurality of fasteners (832).

11. The tissue retractor kit of claim 10, wherein at least one of first plurality of fasteners (812) and the second plurality of fasteners (832) comprise at least one of hooks, clips, clamps, pins, wires, hook-and-loop fasteners, laces and eyelets.

## Patentansprüche

1. Geweberetraktor (1700), der Folgendes umfasst:
eine Längsachse, die einen Instrumentenzugangskanal definiert, der sich von einem proximalen Ende zu einem distalen Ende erstreckt;
einen äußeren Ring (1710);
einen inneren Ring (1730); und
eine flexible Hülse (1750), die sich zwischen dem äußeren Ring (1710) und dem inneren Ring (1730) erstreckt, wobei sich der Instrumentenzugangskanal durch den äußeren Ring (1710), den inneren Ring (1730) und die flexible Hülse (1750) erstreckt, wobei der äußere Ring (1710) konfiguriert ist, um die flexible Hülse (1750) um den äußeren Ring (1710) zu rollen, um die flexible Hülse (1750) mit einer radialen Rückzugskraft bereitzustellen, die ausreicht, um einen Einschnitt zu einem gewünschten Durchmesser zu dehnen, **dadurch gekennzeichnet, dass** die flexible Hülse (1750) eine flexible Metallhülse ist und eines von Folgenden umfasst:
ein Netz, das eine Vielzahl von Metallschlaufen (1752) umfasst, die verknüpft sind,
eine Vielzahl von Metallschlaufen (1752), die nicht miteinander verknüpft sind, sondern durch einen Faden oder Draht verbunden sind, der sich durch benachbarte Schlaufen der Vielzahl von Metallschlaufen (1752) erstreckt, oder
eine Vielzahl von Metallketten.

2. Geweberetraktor (1700) nach Anspruch 1, wobei die flexible Hülse (1750) durchstoßfest ist.

3. Geweberetraktor (1700) nach Anspruch 1, wobei die Vielzahl von Metallschlaufen (1752), die durch den Faden oder Draht verbunden sind, oder die Vielzahl von Metallketten mit dem äußeren Ring (1710) derart verbunden sind, dass der äußere Ring (1710) die Vielzahl von Metallschlaufen (1752) oder die Vielzahl von Metallketten um den äußeren Ring (1710) rollen kann, wenn der äußere Ring (1710) gerollt wird.

4. Geweberetraktor (1700) nach Anspruch 1, wobei die flexible Hülse (1750) wiederverwendbar ist.

5. Geweberetraktor (1700) nach Anspruch 1, wobei die flexible Hülse (1750) lösbar mit dem äußeren Ring (1710) an dem proximalen Ende und dem inneren Ring (1730) an dem distalen Ende verbunden ist.

6. Geweberetraktor (1700) nach Anspruch 1, wobei die flexible Hülse (1750) Edelstahl, Nitinol oder Titan umfasst.

7. Geweberetraktor (1700) nach Anspruch 1, der weiter einen Polymerfilm umfasst, der um die flexible Hülse (1750) angeordnet ist und mit dem äußeren Ring (1710) verbunden ist, wobei der äußere Ring (1710) die flexible Hülse (1750) und den Polymerfilm um den äußeren Ring (1710) rollen kann.

8. Geweberetraktor (1700) nach Anspruch 1, wobei die flexible Hülse (1750) weiter eines oder mehrere eines Kunststoffs, einer Keramik oder eines faserverstärkten Verbundstoffs umfasst.

9. Geweberetraktor (1700) nach Anspruch 1, wobei die flexible Hülse (1750) autoklavierbar ist.

10. Geweberetraktor-Kit für den Geweberetraktor (1700) nach Anspruch 1, der Folgendes umfasst:
den äußeren Ring (1710), der eine erste Vielzahl von Befestigungsmitteln (812) aufweist, die um einen Umfang des äußeren Rings (1710) angeordnet sind;
den inneren Ring (1730), der eine zweite Vielzahl von Befestigungsmitteln (832) aufweist, die um einen Umfang des inneren Rings (1730) angeordnet sind; und
die flexible Metallhülse (1750), die ein distales Ende und ein proximales Ende aufweist,
wobei der Geweberetraktor (1700) zum Zusammenbau durch Befestigen des proximalen Endes der flexiblen Metallhülse (1750) an der ersten Vielzahl von Befestigungsmitteln (812) und des distalen Endes der flexiblen Metallhülse (1750) an der zweiten Vielzahl von Befestigungsmitteln (832) konfiguriert ist.

11. Geweberetraktor-Kit nach Anspruch 10, wobei mindestens eine der ersten Vielzahl von Befestigungsmitteln (812) und der zweiten Vielzahl von Befestigungsmitteln (832) mindestens eines von Haken, Klammern, Klemmen, Stiften, Drähten, Klettverschlüssen, Schnüren und Ösen umfasst.

## Revendications

1. Ecarteur de tissus (1700) comprenant :
un axe longitudinal définissant un canal d'accès pour instrument s'étendant d'une extrémité proximale à une extrémité distale ;
un anneau externe (1710) ;
un anneau interne (1730) ; et
une gaine flexible (1750) s'étendant entre l'anneau externe (1710) et l'anneau interne (1730), dans lequel le canal d'accès pour instrument s'étend à travers l'anneau externe (1710), l'anneau interne (1730) et la gaine flexible (1750), dans lequel l'anneau externe (1710) est configuré pour enrouler la gaine flexible (1750) autour de l'anneau externe (1710) afin de fournir à la gaine flexible (1750) une force d'écartement radial suffisante pour étirer une incision à un diamètre désiré, **caractérisé en ce que** la gaine flexible (1750) est une gaine métallique flexible et est composée de l'un/l'une d'entre :
un maillage comprenant une pluralité de boucles métalliques (1752) qui sont liées,
une pluralité de boucles métalliques (1752) qui ne sont pas entrelacées mais qui sont jointes par un fil en textile ou un fil métallique s'étendant à travers des boucles adjacentes de la pluralité de boucles métalliques (1752) ; ou
une pluralité de chaînes métalliques.

2. Ecarteur de tissus (1700) selon la revendication 1, dans lequel la gaine flexible (1750) est résistante à la perforation.

3. Ecarteur de tissus (1700) selon la revendication 1, dans lequel la pluralité de boucles métalliques (1752) jointes par le fil en textile ou le fil métallique ou la pluralité de chaînes métalliques sont raccordées à l'anneau externe (1710) en ce que l'anneau externe (1710) peut enrouler la pluralité de boucles métalliques (1752) ou la pluralité de chaînes métalliques autour de l'anneau externe (1710) au fur et à mesure que l'anneau externe (1710) roule.

4. Ecarteur de tissus (1700) selon la revendication 1, dans lequel la gaine flexible (1750) est réutilisable.

5. Ecarteur de tissus (1700) selon la revendication 1, dans lequel la gaine flexible (1750) est raccordée de manière détachable à l'anneau flexible (1710) à l'extrémité proximale et à l'anneau interne (1730) à l'extrémité distale.

6. Ecarteur de tissus (1700) selon la revendication 1, dans lequel la gaine flexible (1750) comprend de l'acier inoxydable, du nitinol ou du titane.

7. Ecarteur de tissus (1700) selon la revendication 1, comprenant en outre un film polymère placé autour de la gaine flexible (1750) et raccordé à l'anneau externe (1710) dans lequel l'anneau externe (1710) peut enrouler la gaine flexible (1750) et le film polymère autour de l'anneau externe (1710).

8. Ecarteur de tissus (1700) selon la revendication 1, dans lequel la gaine flexible (1750) comprend en outre un ou plusieurs d'entre un plastique, une céramique ou un composite renforcé fibre.

9. Ecarteur de tissus (1700) selon la revendication 1, dans lequel la gaine flexible (1750) est autoclavable.

10. Kit d'écarteur de tissus pour l'écarteur de tissus (1700) selon la revendication 1, comprenant :
l'anneau externe (1710) ayant une première pluralité de dispositifs de fixation (812) placés autour d'une circonférence de l'anneau externe (1710) ;
l'anneau interne (1730) ayant une deuxième pluralité de dispositifs de fixation (832) placés autour d'une circonférence de l'anneau interne (1730) ; et
la gaine métallique flexible (1750) ayant une extrémité distale et une extrémité proximale,
dans lequel l'écarteur de tissus (1700) est configuré pour être assemblé en fixant l'extrémité proximale de la gaine métallique flexible (1750) à la première pluralité de dispositifs de fixation (812) et l'extrémité distale de la gaine métallique flexible (1750) à la deuxième pluralité de dispositifs de fixation (832).

11. Kit d'écarteur de tissus selon la revendication 10, dans lequel au moins l'une de la première pluralité de dispositifs de fixation (812) et de la deuxième pluralité de dispositifs de fixation (832) comprend au moins l'un d'entre des crochets, des pinces, des clamps, des épingles, des fils métalliques, des bandes autoagrippantes, des lacets et des œillets.
